# EUROPÄISCHE PATENTSCHRIFT

(11) **EP 0 859 592 B1**
(45) Veröffentlichungstag und Bekanntmachung des Hinweises auf die Patenterteilung: **28.05.2003**
(21) Anmeldenummer: 96934520.6
(22) Anmeldetag: 02.10.1996
(51) Int. Cl.: A61K 7/48, A61P 17/16

(54) **HAUTPFLEGEMITTEL FÜR GEALTERTE HAUT**
SKIN-CARE AGENT FOR AGEING SKIN
PRODUIT DE SOIN POUR LA SENESCENCE CUTANEE

(30) Priorität: 05.10.1995 DE 19537027
(43) Veröffentlichungstag der Anmeldung: 26.08.1998
(73) Patentinhaber: Beiersdorf Aktiengesellschaft, 20245 Hamburg (DE)
(72) Erfinder: HOPPE, Udo, D-22397 Hamburg (DE); SAUERMANN, Gerhard, D-24649 Wiemersdorf (DE); SCHREINER, Volker, D-20259 Hamburg (DE); STÄB, Franz, D-21379 Echem (DE)
(86) Internationale Anmeldenummer: EP9604302
(87) Internationale Veröffentlichungsnummer: WO97012591

(56) Entgegenhaltungen:
- DE-A- 4 327 063
- FR-A- 2 540 381
- US-A- 5 378 461
- PATENT ABSTRACTS OF JAPAN vol. 10, no. 178 (C-355) [2234] , 21.Juni 1986 & JP 61 027914 A (SHISEIDO), 7.Februar 1986,

## Beschreibung

Die Haut altert bedingt durch endogene, genetisch determinierte Einflüsse. Exogene Faktoren, wie UV-Licht und chemische Noxen, können kumulativ wirksam sein und beschleunigen die natürlichen Alterungsprozesse. Es kommt dadurch zu zahlreichen degenerativen Prozessen, die je nach Größe der Einflußfaktoren u.a. zu folgenden strukturellen Veränderungen und Schäden in Dermis und Epidermis führen (z.B. auch der Dermatoheliosis):
a) Rückbildung des mikrovasculären Systems.
b) Schlaffheit und Ausbildung von Falten teilweise aufgrund einer Abnahme und Quervernetzung des Kollagens, Akkumulation der Glucosaminoglycane (Grundsubstanz) und solarer Elastosis (Elastinverklumpung).
c) Abflachung der Retezapfen. Damit verbunden ist die Verringerung der Fläche zwischen Dermis und Epidermis, über die Stoffe zur Ernährung und Entschlakkung der Epidermis ausgetauscht werden.
d) Eingeschränkter regenerativer Turnover in der Epidermis, verbunden mit fehlerhafter Ausbildung der Hornschicht (Verhornungsstörungen), die zur Hautaustrocknung und zu Hautrauhigkeit und Hautrissigkeit führt.
e) Fehlerhafte Regulation von Zellteilung (Profileration) und Zellreifung (Differenzierung) in der Epidermis, woraus zelluläre Atypen und Atrophien und der Verlust der Polarität resultieren,
f) Lokale Hyper-, Hypo- und Fehlpigmentierungen (Altersflecken).

Die vorliegende Erfindung betrifft Produkte zur Pflege und Prophylaxe bei Altershaut, insbesondere lichtgealterter Haut und durch endogene Mechanismen chronologisch gealterten Haut sowie zur Behandlung der Folgeschäden der Lichtalterung und endogen bedingten Alterung der Haut, insbesondere der unter a) bis f) aufgeführten Phänomene und bevorzugt zur Behandlung und prophylaktischen Behandlung von Falten und Hautrauhigkeit.

Produkte zur Pflege, Prophylaxe und Behandlung lichtgealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden bei Lichtalterung ist allerdings umfangmäßig begrenzt. Die Verwendung Vitamin A-Säure-haltiger Produkte bedingt darüber hinaus oft starke erythematöse Hautreizungen.

Weiterhin sind kosmetische Zubereitungen mit Coenzym Q-10 aus der DE-A- 33 09 850 bekannt, die zur Behandlung von Hautkrankheiten, zur Prophylaxe von dystrophischen und dysmetabolischen Zuständen der Haut und zur Anwendung bei chemischen und physikalischen Respirationsschäden oder bei verzögerter Respiration verbunden mit Alter und Abnutzung geeignet sind.

In der japanischen Offenlegungsschrift 58,180,410 ist die Eignung von Coenzym Q-10 für Kosmetika beschrieben. Es soll den Hautzellmetabolismus aktivieren und die Oxidation unterdrücken. Coenzym Q-10 hat im Resultat eine wichtige Funktion bei der Prävention von Hautschäden durch UV-Strahlen und der Prävention von Hautalterung. Bei 20 bis 40-jährigen wird die Hautrauhigkeit gebessert, indem der Haut Feuchtigkeit gegeben wird.

Ziel der vorliegenden Erfindung war es somit, Wege zu finden, die Nachteile des Standes der Technik zu vermeiden. Insbesondere sollte die prophylaktische Wirkung und die restrukturierende Wirkung bei Hautalterung dauerhaft, nachhaltig und ohne das Risiko von Nebenwirkungen sein.

Erfindungsgemäß werden diese Ziele von Hautpflegepräparaten und Wirkstoffkombinationen erreicht, in denen Sterine in Kombination mit Ubichinonen und/oder Plastochinonen enthalten sind.

Gut geeignete Sterine (auch als "Sterole" bezeichnet) sind z.B. Zoosterine, Phytosterine und Mykosterine.

Bevorzugte Zoosterine sind Cholesterin, Dihydrocholesterin, 7-Dehydrocholesterin, Lanosterin, Dihydrolanosterin, Spongosterin und Stellasterin.

Bevorzugte Phytosterine sind Ergosterin, Sitosterin, Stigmasterin, Fucosterin, Brassicasterin und Campesterin.

Bevorzugte Mykosterine sind Ergosterin, Fungisterin und Zymosterin.

Biochemische Vorstufen der Sterine sind Mevalonsäure, Farnesol und Squalen.

Bevorzugte Verbindungen der Gruppe (A) sind insbesondere Cholesterin, 7-Dehydrocholesterin, Dihydrocholesterin, Lanosterin, Dihydrolanosterin, Spongosterin und Stellasterin.

Bevorzugt werden Zubereitungen mit einem Gehalt an einer Verbindung oder zwei Verbindungen oder drei Verbindungen, ausgewählt aus der Gruppe (A), kombiniert mit einer Verbindung oder zwei Verbindungen oder drei Verbindungen aus der Gruppe (B).

Die erfindungsgemäßen topischen Zubereitungen können kosmetische oder dermatologische Zubereitungen sein. Sie dienen, wie auch die Wirkstoffe, zur Pflege und Prophylaxe bei Lichtalterung und chronologischer Hautalterung und zur Behandlung lichtgealterter Haut und chronologisch gealterter Haut.

Gegenstand der Erfindung ist die Verwendung von einer Verbindung oder mehreren Verbindungen ausgewählt aus der Gruppe (A), bestehend aus Sterinen in Kombination mit einer Verbindung oder mehreren Verbindungen, ausgewählt aus der Gruppe (B), bestehend aus Ubichinonen und deren Derivaten und Plastochinonen und deren Derivaten zur Herstellung von topischen Zubereitungen zur Pflege und Prophylaxe bei Lichtalterung und chronologischer Hautalterung und zur Behandlung lichtgealterter Haut und chronologisch gealterter Haut.

Die erfindungsgemäßen Wirkstoffkombinationen und die damit erhältlichen Zubereitungen bewirken die Restrukturierung der durch lichtbedingte Einflüsse gealterten Haut oder der durch endogene Mechanismen chronologisch gealterten Haut, insbesondere der faltigen, rauhen, trockenen, rissigen und/oder schuppigen Haut.

Gegenstand der Erfindung ist also auch die Verwendung der vorstehenden Zubereitungen und Wirkstoffe für die beschriebenen Zwecke, vorzugsweise aber die Verwendung zur Prophylaxe und Behandlung der folgenden Erscheinungen a) bis f), insbesondere der Dermatoheliosis und der chronolgisch gealterten Haut:
a) Rückbildung des mikrovasculären Systems.
b) Schlaffheit und Ausbildung von Falten teilweise aufgrund einer Abnahme und Quervernetzung des Kollagens, Akkumulation der Glucosaminoglycane (Grundsubstanz) und solarer Elastosis (Elastinverklumpung).
c) Abflachung der Retezapfen. Damit verbunden ist die Verringerung der Fläche zwischen Dermis und Epidermis, über die Stoffe zur Ernährung und Entschlakkung der Epidermis ausgetauscht werden.
d) Eingeschränkter regenerativer Turnover in der Epidermis, verbunden mit fehlerhafter Ausbildung der Hornschicht (Verhornungsstörungen), die zur Hautaustrocknung und zur Hautrauhigkeit und Hautrissigkeit und Schuppigkeit führt.
e) Fehlerhafte Regulation von Zellteilung (Profileration) und Zellreifung (Differenzierung) in der Epidermis, woraus zelluläre Atypen und Atrophien und der Verlust der Polarität resultieren.
f) Lokale Hyper-, Hypo- und Fehlpigmentierungen (Altersflecken).

Besonders bevorzugt werden die Kombinationen von Cholesterin mit Ubichinonen, insbesondere Coenzym Q-6 oder Q-9 oder Q-10, insbesondere aber die Kombinationen von Cholesterin mit Coenzym Q-10, und Zubereitungen damit und die Verwendungen für die vorstehenden Zwecke.

Ubichinone (auch Coenzyme Qₙ) sind eine Gruppe von Substanzen, die an ihrem Chinonring n Isopren-Einheiten kettenförmig gebunden haben (Q₀ - Q₁₀). Ubichinone fungieren bei der biologischen, mitochondralen Oxidation als Elektronenüberträger und spielen so eine bedeutende Rolle beim Energiestoffwechsel der Zellen. Plastochinone sind analoge Verbindungen aus dem Pflanzenbereich, die bei der Photosynthese eine Rolle spielen. In kosmetischen Zubereitungen finden Ubichinone seit langem Anwendung als Antioxidantien zum Schutz oxidationsempfindlicher Substanzen vor Sauerstoff-Radikal-induziertem Zerfall.

Mit "Ubichinone" und "Plastochinone" sind hier auch Ubichinone und deren Derivate" und "Plastochinone und deren Derivate" gemeint.

Die Ubichinone sind aus der Literatur bekannt (z.B. "Römpp Chemie Lexikon", Georg Thieme Verlag Stuttgart, New York, 9. Auflage, S. 4784-4785, oder "The Merck Index", 11th Edition, Merck & Co., Inc. Rahway, N.Y., USA, Abstr. 9751 (1989)). Sie werden auch als Mitochinone oder Coenzyme Q bezeichnet. Die Anzahl der Isopren-Einheiten in der Seitenkette wird mit n in der Bezeichnung Coenzyme Q-n angegeben, worin n eine ganze Zahl bedeutet. Bevorzugt werden Ubichinone oder Coenzyme Q-n mit n=0-12, besonders bevorzugt n=1-12 und insbesondere n=6 bis 10. Gegenstand der Erfindung ist also auch der Chinongrundkörper des Ubichinons ohne Isoprensubstituenten. Erfindungsgemäße Ubichinone oder deren Derivate sind z.B. auch Alkyl-Ubichinone, insbesondere 6-Alkyl-Ubichinone mit vorzugsweise C₁-C₁₂-Alk yl-Resten. Bevorzugt wird Decyl-Ubichinon, insbesondere 6-Decyl-Ubichinon oder 2,3-Dimethoxy-5-methyl-6-decyl-1,4-benzochinon.

Die Plastochinone sind ebenfalls aus der Literatur bekannt (z.B. "Römpp Chemie Lexikon", Georg Thieme Verlag, Stuttgart, New York, 9. Auflage, S. 3477). Sie sind in der Struktur eng mit den Ubichinonen verwandt und zählen auch zu den Isoprenoid-Chinonen, da sie am Chinonring eine Seitenkette aus Isopren-Einheiten tragen. Bevorzugt werden Plastochinone mit 0 - 12, besonders bevorzugt 1 - 10 und insbesondere 6 bis 10 Isopren-Einheiten in der Seitenkette. Gegenstand der Erfindung ist also auch der Chinongrundkörper des Plastochinons ohne Isoprensubstituenten.

Erfindungsgemäße Plastochinone oder deren Derivate sind z.B. auch Alkyl-Plastochinone mit vorzugsweise C₁-C₁₂-Alkyl-Resten. Bevorzugt werden Decyl-Plastochinone, insbesondere 5- oder 6-Decyl-Plastochinon oder 2,3-Dimethyl-5-decyl-1,4-benzochinon.

Ubichinone fungieren bei der biologischen, mitochondralen Oxidation als Elektronenüberträger und spielen so eine bedeutende Rolle beim Energiestoffwechsel der tierischen Zellen. In kosmetischen Zubereitungen finden Ubichinone seit langem Anwendung als Antioxidantien zum Schutz oxidationempfindlicher Substanzen.

Plastochinone sind analoge Verbindungen aus dem Pflanzenbereich, die bei der Photosynthese in den Chloroplasten der pflanzlichen Zellen eine Rolle spielen.

Sie unterscheiden sich von den Ubichinonen in drei Substituenten am Chinonring, wobei die zwei Methoxy-Gruppen in den Ubichinonen durch Methylgruppen und eine Methylgruppe durch ein Wasserstoffatom ersetzt sind. Strukturgleich sind jedoch die kettenförmig gebundenen Isopren-Einheiten (vergl. z.B. Pfister und Arntzen, Z. für Naturforschung C34; 996ff, 1979).

Besonders bevorzugt werden die folgenden erfindungsgemäßen Wirkstoffe und Kombinationen damit:
Coenzym Q - 10, Coenzym Q - 9, Coenzym Q - 8, Coenzym Q - 7, Coenzym Q - 6,
und
Plastochinon mit 10 Isopreneinheiten (auch PQ-10 genannt entsprechend der IUB-Abkürzung PQ für Plastochinone, in der Formel PQ-n soll n die Anzahl der Isopren-Einheiten (0 bis 12) angeben), PQ-9, PQ-8, PQ-7, PQ-6.

Es hat sich überraschenderweise gezeigt, daß Sterine in Kombination mit Ubichinonen und/oder Plastochinonen beim Schutz vor Lichtalterung und chronologischer Hautalterung bzw. bei der Reparatur von lichtbedingten und endogen bedingten Strukturschäden der Haut in synergistischer Weise zusammenwirken, was in signifikanter Weise dem Nachteil des Standes der Technik abhilft.

Die Konzentrationen der Sterine in topischen Zubereitungen liegen bevorzugt zwischen 0.01 und 99 Gew.-%.

Die Konzentrationen von Ubichinonen und/oder Plastochinonen in topischen Zubereitungen liegen bevorzugt zwischen 0.001 und 99 Gew.-%.

Vorteilhaft enthalten erfindungsgemäße Zubereitungen, Hautpflegeprodukte oder Dermatika Kombinationen in folgender Zusammenstellung:
0,01 - 10 Gew.-% Sterine und deren biochemische Vorstufen und
0,001 - 10 Gew.-% Ubichinon und/oder Plastochinon

Bevorzugt enthalten topische Zubereitungen oder Dermatika
0,05 - 1 Gew.-% Cholesterin und
0,05 - 1 Gew.-% Coenzym Q₁₀

Ganz besonders bevorzugt enthalten die Zubereitungen oder Dermatika
0,4 Gew.-% Cholesterin und
0,4 Gew.-% Coenzym Q₁₀

Im Rahmen der Anmeldung sind stets Gewichtsprozente bezogen auf 100 Gew.-% Gesamtzusammensetzung der jeweiligen erfindungsgemäßen Zubereitungen oder Dermatikums gemeint.

Die erfindungsgemäßen Wirkstoffkombinationen oder Wirkstoffe können in den topischen Zubereitungen in Mengen von 0,001 bis 99 Gew.-%, z.B. auch in Mengen von 0,001 bis 50 Gew.-% vorliegen, jeweils bezogen auf das Gesamtgewicht der Zubereitungen.

Vorzugsweise können die erfindungsgemäßen Wirkstoffkombinationen oder Wirkstoffe in den topischen Zubereitungen in Mengen von 0,01 bis 10 Gew.-%, insbesondere in Mengen von 0,1 bis 1 Gew.-% vorliegen, jeweils bezogen auf das Gesamtgewicht der Zubereitungen.

In den Kombinationen können die Gewichtsverhältnisse der beiden Komponenten in großen Bereichen schwanken, z.B. im Verhältnis 1 : 100 bis 100 : 1, vorzugsweise im Verhältnis 1 : 10 bis 10 : 1. Sie können z.B. auch im Gewichtsverhältnis 1 : 2 bis 2 : 1 oder 1 : 1 vorliegen.

Erfindungsgemäße topische Zubereitungen oder Zusammensetzungen mit den erfindungsgemäßen Kombinationen und Wirkstoffen sind alle gängigen Anwendungsformen. z.B. Cremes (W/O, O/W, W/O/W), Gele, Lotionen, Milchen.

Die erfindungsgemäßen topischen Zubereitungen können als flüssige, pastöse oder feste Zubereitungen formuliert werden, beispielsweise als wäßrige oder alkoholische Lösungen, wäßrige Suspensionen, Emulsionen, Salben, Cremes, Öle, Pulver oder Stifte. In Abhängigkeit von der gewünschten Formulierung können die Wirkstoffe in pharmazeutische und kosmetische Grundlagen für topische Applikationen eingearbeitet werden, die als weitere Komponenten beispielsweise Ölkomponenten, Fett und Wachse, Emulgatoren, anionische, kationische, ampholyitsche, zwitterionische und/oder nichtionogene Tenside, niedere ein- und mehrwertige Alkohole, Wasser, Konservierungsmittel, Puffersubstanzen, Verdikkungsmittel, Duftstoffe, Farbstoffe und Trübungsmittel enthalten. Vorteilhaft können die erfindungsgemäßen Wirkstoffe auch in transdermalen therapeutischen Systemen, insbesondere kubischen Systemen verwendet werden.

Es ist ferner von Vorteil, den Zubereitungen Antioxidantien (z.B. alpha-Tocopherol, Vitamin E und C, Flavone, Flavonoide, Imidazole, alpha-Hydroxycarbonsäuren (z.B. Äpfelsäure, Glycolsäure, Gluconsäure, Salicylsäure sowie deren Derivate) und/oder Eisenkomplexbildner (z.B. EDTA, alpha-Hydroxyfettsäuren) und/oder bekannte UV-Lichtschutzfilter in Mengen von z.B. 0,1 bis 10 Gewichtsprozenten zuzusetzen, um die Stabilität der oxidationsempfindlichen Wirkstoffe zu gewährleisten.

Auch ist es vorteilhaft, den Zubereitungen insbesondere 0,01 - 10 Gewichtsprozente an Stoffen bzw. Stoffkombinationen des aeroben zellulären Energiestoffwechsels (z.B. zelluläre Energieüberträger (wie Kreatin, Guanin, Guanosin, Adenin, Adenosin, Nicotin, Nicotinamid, Riboflavin), Coenzyme (z.B. Pantothensäure, Panthenol, Liponsäure, Niacin), Hilfsfaktoren (z.B. L-Carnitin, Dolichol, Uridin), Substrate (z.B. Hexosen, Pentosen, Fettsäuren) und intermediäre Stoffwechselprodukte (z.B. Zitronensäure, Pyruvat) und/oder Glutathion zuzusetzen.

Es ist weiterhin vorteilhaft, den Zubereitungen Penetrationsförderer zuzusetzen, insbesondere Ölsäure, cis-6-Hexadecensäure oder Palmitoleinsäure. Penetrationsförderer können in Mengen von 0,01 Gew.-% bis 1,0 Gew.-% in den Zubereitungen enthalten sein.

Es ist weiterhin vorteilhaft, den Zubereitungen Ceramide zuzusetzen. Sie können in Mengen von 0,01 Gew.-% bis 5,0 Gew.-% in den Zubereitungen enthalten sein.

Vorteilhaft können erfindungsgemäße Zubereitungen außerdem Substanzen enthalten, die UV-Strahlung im UVA- und/oder im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel für die Haut dienen. In den Zubereitungen wirken die UV-Absorber gegenüber den Wirkstoffen als Antioxidantien.

Enthalten die erfindunsgemäßen Emulsionen UVB-Filtersubstanzen, können diese öllöslich oder wasserlöslich sein. Erfindungsgemäß vorteilhafte öllösliche UVB-Filter sind z.B.:
3-Benzylidencampher-Derivate, vorzugsweise 3-(4-Methylbenzyliden)campher, 3-Benzylidencampher.

Vorteilhaft wasserlösliche UVB-Filter sind z.B.:
Salze der 2-Phenylbenzimidazol-5-sulfonsäure wie ihr Natrium-, Kalium- oder ihr Triethanolammonium-Salz, sowie die Sulfonsäure selbst.

Es kann auch von Vorteil sein, erfindungsgemäße Wirkstoffkombinationen mit UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'isopropylphenyl)propan-1,3dion. Auch diese Kombinationen bzw. Zubereitungen, die diese Kombinationen enthalten, sind Gegenstand der Erfindung. Es können die für die UVB-Kombination verwendeten Mengen eingesetzt werden.

Gegenstand der Erfindung sind also auch die Kombinationen der erfindungsgemäßen Verwendung, insbesondere in den topischen Zubereitungen, mit Antioxydantien, Stoffen des aeroben zellulären Energiestoffwechsels und/oder UV-Absorbern, durch die sich z.B. die Stabilität und die Wirkung der Zubereitung verbessern läßt.

Die vorstehend aufgeführten Beispiele für kombinierbare Wirkstoffe aus den angegebenen Wirkstoffgruppen dienen dazu, die Erfindung zu beschreiben, ohne daß beabsichtigt ist, die Erfindung auf diese Beispiele zu beschränken.

Darüber hinaus können schützende Formulierungsformen angewendet werden, wobei die erfindungsgemäßen Stoffe z.B. in Liposomen, Micellen, Nanosphären usw. aus z.B. hydrierten Amphiphilen, wie z.B. Ceramiden, Fettsäuren, Sphingomyelin und Phosphoglyceriden bzw. in Zyklodextrane eingeschossen (verkapselt) werden. Weiterer Schutz kann durch die Verwendung von Schutzgas (z.B. N₂, CO₂) bei der Formulierung und die Verwendung gasdichter Verpakkungsformen erreicht werden.

Weitere Hilfs- und Zusatzstoffe können wasserbindende Stoffe, Verdicker, Füllstoffe, Parfüm, Farbstoffe, Emulgatoren, Wirkstoffe wie Vitamine, Konservierungsmittel, Wasser und/oder Salze sein.

Bei der Verarbeitung der Wirkstoffe und anderer oxidationsempfindlicher Stoffe sollte die Temperatur nicht über 40°C liegen. Ansonsten sind die üblichen Maßregeln zu beachten, die dem Fachmann bekannt sind.

Die erfindungsgemäßen Stoffgruppen lassen sich so in alle kosmetischen Grundlagen einarbeiten. Grundsätzlich sind allerdings W/O- und O/W-und W/O/W-Emulsionen bevorzugt. Besonders vorteilhaft können erfindungsgemäße Kombinationen in Pflegeprodukte wie beispielsweise O/W-Cremes, W/O-Cremes, O/W-Lotionen, W/O-Lotionen usw. eingesetzt werden.

Alle Mengenangaben, Prozentangaben oder Teile beziehen sich, soweit nicht anders angegeben, auf das Gewicht, insbesondere auf das Gesamtgewicht der Zubereitungen oder der jeweiligen Mischung.

Die folgenden Beispiele dienen dazu, die Erfindung zu beschreiben, ohne daß beabsichtigt ist, die Erfindung auf diese Beispiele zu beschränken.

| Kombination | A | B | C | D | E | F |
|---|---|---|---|---|---|---|
| Cholesterin | 50 | 50 | 10 | 90 | 60 | 50 |
| Coenzym Q₉ | - | 25 | - | - | - | 40 |
| Coenzym Q₁₀ | 50 | 25 | 90 | 10 | 40 | 10 |
| | 100 | 100 | 100 | 100 | 100 | 100 |

Die Teile und Zahlenangaben beziehen sich auf Gewichtsteile.

### Beispiel I

### (mit Kombination A)

| Hautcreme vom W/O-Typ | |
|---|---|
| | Gew.-Teile |
| Vaseline DAB 9 | 13 |
| Glycerin DAB 9 | 6,3 |
| Wasser VES | 34,4 |
| Paraffinöl (Mineralöl 5E,Shell) | 31 |
| Cetearylalkohol/PEG-40-Castor Oil/Natriumcetearyl-sulfat (Emulgade F, Henkel KGaA) | 2,5 |

In die 75°C warme Fettphase werden 0,3 Teile Coenzym Q₁₀ in 3 Teilen Paraffinöl gelöst eingearbeitet. Die Fettphase wir sodann zu der 75°C warmen Wasserphase gegeben, verrührt und homogenisiert, bis eine gleichmäßige hellgelbe Creme entstanden ist. 0,3 Teile Cholesterin werden bei Raumtemperatur in weiteren 3,2 Teilen Paraffinöl gelöst und in die abgekühlte Creme eingerührt.

Beispiel I hat folgende endgültige Zusammensetzung:

| | Gew.-Teile |
|---|---|
| Vaseline DAB 9 | 13 |
| Glycerin DAB 9 | 6,3 |
| Wasser VES | 34,4 |
| Paraffinöl (Mineralöl 5E,Shell) | 43,2 |
| Cetearylalkohol/PEG-40-Castor Oil/Natriumcetearyl-sulfat (Emulgade F, Henkel KGaA) | 2,5 |
| Cholesterin | 0,3 |
| Coenzym Q₁₀ | 0,3 |
| | 100 |

### Beispiel II

### (mit Kombination B)

| Hautcreme vom W/O-Typ | |
|---|---|
| | Gew.-Teile |
| PEG-1 Glyceryl-Oleostearat + Paraffinwachs | 8 |
| Vaseline DAB | 2,8 |
| Paraffinwachs/Paraffin | 1,8 |
| Ceresin | 2,2 |
| Octyldodecanol (Eutanol G, Henkel KGaA) | 10 |
| Propylenglycol | 1 |
| Glycerin | 1 |
| Magnesiumsulfat | 0,7 |
| Wasser VES (vollentsalzt) | 59,7 |
| Summe Additive (Parfüm, Konservierung, Stabilisation) | 0,8 |

In die 75°C warme Fettphase werden 0,2 Teile Coenzym Q₁₀ und 0,2 Teile Coenzym Q₉ in 6 Teilen Paraffinöl gelöst eingearbeitet. Die Fettphase wird sodann zu der 75°C warmen Wasserphase gegeben, verrührt und homogenisiert, bis eine gleichmäßige hellgelbe Creme entstanden ist. 0,4 Teile Cholesterin werden bei Raumtemperatur in weiteren 5,5 Teilen Paraffinöl gelöst und in die abgekühlte Creme eingerührt.

Beispiel II hat folgende endgültige Zusammensetzung:

| | Gew.-Teile |
|---|---|
| PEG-1 Glyceryl-Oleostearat + Paraffinwachs | 8 |
| Vaseline DAB | 2,8 |
| Paraffinwachs/Paraffin | 1,8 |
| Paraffinöl (Mineralöl 5E,Shell) | 11,5 |
| Ceresin | 2,2 |
| Octyldodecanol | 10 |
| Cholesterin | 0,4 |
| Coenzym Q₉ | 0,2 |
| Coenzym Q10 | 0,2 |
| Propylenglycol | 1 |
| Glycerin | 1 |
| Magnesiumsulfat | 0,7 |
| Wasser VES | 59,4 |
| Summe Additive (Parfüm, Konservierung, Stabilisation) | 0,8 |
| | 100 |

### Beispiel III

### (mit Kombination C)

| Hautcreme vom O/W-Typ | |
|---|---|
| | Gew.-Teile |
| Octyldodecanol (Eutanol G, Henkel KGaA) | 9,3 |
| Cetearylalkohol/PEG-40-Castor Oil/Natriumcetearylsulfat (Emulgade F, Henkel KGaA) | 3,7 |
| Wasser VES | 73,7 |
| Glycerin DAB 9 | 4,6 |

In die 75°C warme Fettphase werden 0,9 Teile Coenzym Q₁₀ in 4 Teilen Paraffinöl gelöst eingearbeitet. Die Fettphase wir sodann zu der 75°C warmen Wasserphase gegeben, verrührt und homogenisiert, bis eine gleichmäßige hellgelbe Creme entstanden ist. 0,1 Teile Cholesterin werden bei Raumtemperatur in weiteren 3,7 Teilen Paraffinöl gelöst und in die abgekühlte Creme eingerührt.

Beispiel III hat folgende entgültige Zusammensetzung:

| | |
|---|---|
| Octyldodecanol (Eutanol G, Henkel KGaA) | 9,3 |
| Cetearylalkohol/PEG-40-Castor Oil/Natriumcetearylsulfat (Emulgade F, Henkel KGaA) | 3,7 |
| Wasser VES | 73,7 |
| Glycerin DAB 9 | 4,6 |
| Paraffinöl (Mineralöl 5E, Shell) | 7,7 |
| Coenzym Q₁₀ | 0,9 |
| Cholesterin | 0,1 |
| | 100 |

### Beispiel IV

### (mit Kombination D)

| O/W-Lotion | |
|---|---|
| | Gew.-Teile |
| Steareth-2 | 3 |
| Steareth-21 | 2 |
| Cetearylalkohol/PEG-40-Castor Oil/Natriumcetearylsulfat (Emulgade F, Henkel KGaA) | 2,5 |
| Propylenglycol | 1 |
| Glycerin | 1 |
| Wasser VES | 74,3 |
| Summe Additive (Parfüm, Konservierung, Stabilisation) | 0,8 |

In die 75°C warme Fettphase werden 0,1 Teile Coenzym Q₁₀ in 5,2 Teile Paraffinöl gelöst eingearbeitet. Die Fettphase wir sodann zu der 75°C warmen Wasserphase gegeben, verrührt und homogenisiert, bis eine gleichmäßige hellgelbe Lotion entstanden ist. 0,9 Teile Cholesterin werden bei Raumtemperatur in weiteren 9 Teilen Paraffinöl gelöst und in die abgekühlte Lotion eingerührt.

Beispiel IV hat folgende endgültige Zusammensetzung:

| | Gew.-Teile |
|---|---|
| Steareth-2 | 3 |
| Steareth-21 | 2 |
| Cetearylalkohol/PEG-40-Castor Oil/Natriumcetearylsulfat (Emulgade F, Henkel KGaA) | 2,5 |
| Paraffinöl (Mineralöl 5E, Shell) | 14,4 |
| Propylenglycol | 1 |
| Coenzym Q₁₀ | 0,1 |
| Cholesterin | 0,9 |
| Glycerin | 1 |
| Wasser VES | 74,3 |
| Summe Additive (Parfüm, Konservierung, Stabilisation) | 0,8 |
| | 100 |

### Beispiel V

### (mit Kombination E)

| O/W-Lotion | |
|---|---|
| | Gew.-Teile |
| Octyldodecanol (Eutanol G, Henkel KGaA) | 5,6 |
| Cetearylalkohol/PEG-40-Castor Oil/Natriumcetearylsulfat (Emulgade F, Henkel KGaA) | 8,9 |
| Cetearylisononanoat (Cetiol 5N, Henkel KGaA) | 7,5 |
| Wasser VES | 62,3 |
| Glycerin DAB 9 | 4,7 |

In die 75°C warme Fettphase werden 0,4 Teile Coenzym Q₁₀ in 6 Teilen Paraffinöl gelöst eingearbeitet. Die Fettphase wir sodann zu der 75°C warmen Wasserphase gegeben, verrührt und homogenisiert, bis eine gleichmäßige hellgelbe Lotion entstanden ist. 0,6 Teile Cholesterin werden bei Raumtemperatur in weiteren 4 Teilen Paraffinöl gelöst und in die abgekühlte Lotion eingerührt.

Beispiel V hat folgende endgültige Zusammensetzung:

| | Gew.-Teile |
|---|---|
| Octyldodecanol (Eutanol G, Henkel KGaA) | 5,6 |
| Cetearylalkohol/TEG-40-Castor Oil/Natriumcetearylsulfat (Emulgade F, Henkel KGaA) | 8,9 |
| Cetearylisononanoat (Cetiol 5N, Henkel KGaA) | 7,5 |
| Wasser VES | 62,3 |
| Glycerin DAB 9 | 4,7 |
| Paraffinöl (Mineralöl 5E, Shell) | 10 |
| Coenzym Q₁₀ | 0,4 |
| Cholesterin | 0,6 |
| | 100 |

### Beispiel VI

### (mit Kombination F)

| Hautöl | |
|---|---|
| | Gew.-Teile |
| Glyceryltricaprylat (Miglyol 812, Dynamit Nobel) | 21 |
| Hexyllaurat (Cetiol A, Henkel KGaA) | 20 |
| Octylstearat (Cetiol 886, Henkel KGaA | 20 |
| Paraffinöl (Mineralöl 5E, Shell) | 35 |
| Cholesterin | 2 |
| Coenzym Q₉ | 1,6 |
| Coenzym Q₁₀ | 0,4 |
| | 100 |

Die Komponenten werden bei 25°C verrührt, bis eine gleichmäßige, klare Mischung entstanden ist.

## Patentansprüche

1. Verwendung von einer Verbindung oder mehreren Verbindungen ausgewählt aus der Gruppe (A), bestehend aus Sterinen in Kombination mit einer Verbindung oder mehreren Verbindungen, ausgewählt aus der Gruppe (B), bestehend aus Ubichinonen und deren Derivaten und Plastochinonen und deren Derivaten zur Herstellung von topischen Zubereitungen zur Pflege und Prophylaxe bei Lichtalterung und chronologischer Hautalterung und zur Behandlung lichtgealteter Haut und chronologisch gealterter Haut.

2. Verwendung gemäß Anspruch 1, **dadurch gekennzeichnet, daß** Zoosterine verwendet werden.

3. Verwendung gemäß Anspruch 1, **dadurch gekennzeichnet, daß** mindestens ein Ubichinon verwendet wird.

4. Verwendung gemäß Anspruch 1, **dadurch gekennzeichnet, daß** Cholesterin verwendet wird.

5. Verwendung gemäß Anspruch 1, **dadurch gekennzeichnet, daß** Ubichinone oder Plastochinone 0 bis 12 Isopreneinheiten und gegebenenfalls Alkyl-Resten verwendet werden.

6. Verwendung gemäß Anspruch 1, **dadurch gekennzeichnet, daß** Ubichinone oder Plastochinone mit 9 oder 10 Isopreneinheiten verwendet werden.

7. Verwendung gemäß Anspruch 1, **dadurch gekennzeichnet, daß** Antioxidantien, Stoffe des aeroben zellulären Energiestoffwechsels und/oder UV-Absorber mit verwendet werden.

8. Verwendung gemäß Anspruch 1, **dadurch gekennzeichnet, daß** die topischen Zubereitungen kosmetische oder dermatologische Zubereitungen, insbesondere Öle, W/O-, O/W- oder W/O/W-Emulsionen sind.

## Claims

1. Use of one or more compounds chosen from the group (A) consisting of sterols in combination with one or more compounds chosen from the group (B) consisting of ubiquinones and derivatives thereof and plastoquinones and derivatives thereof for the preparation of topical preparations for the care and prophylaxis in cases of photo-ageing and chronological skin ageing and for the treatment of photo-aged skin and chronologically aged skin.

2. Use according to Claim 1, **characterized in that** zoosterols are used.

3. Use according to Claim 1, **characterized in that** at least one ubiquinone is used.

4. Use according to Claim 1, **characterized in that** cholesterol is used.

5. Use according to Claim 1, **characterized in that** ubiquinones or plastoquinones having from 0 to 12 isoprene units and optionally alkyl radicals are used.

6. Use according to Claim 1, **characterized in that** ubiquinones or plastoquinones having 9 or 10 isoprene units are used.

7. Use according to Claim 1, **characterized in that** antioxidants, substances of aerobic cellular energy metabolism and/or UV absorbers are also used.

8. Use according to Claim 1, **characterized in that** the topical formulations are cosmetic or dermatological formulations, in particular oils, W/O, O/W or W/O/W emulsions.

## Revendications

1. Utilisation d'un ou de plusieurs composés choisis parmi le groupe (A) constitué de stérols en combinaison avec un composé ou plusieurs composés choisis parmi le groupe (B) constitué d'ubiquinones et de leurs dérivés et de plastoquinones et de leurs dérivés, pour la préparation de formulations topiques destinées aux soins et à la prophylaxie dans le cas d'un vieillissement par le soleil et d'un vieillissement cutané chronologique et destinés au traitement d'une peau vieillie par le soleil et d'une peau de vieillissement chronologique.

2. Utilisation selon la revendication 1, **caractérisée en ce que** l'on utilise des zoostérols.

3. Utilisation selon la revendication 1, **caractérisée en ce que** l'on utilise au moins une ubiquinone.

4. Utilisation selon la revendication 1, **caractérisée en ce que** l'on utilise du cholestérol.

5. Utilisation selon la revendication 1, **caractérisée en ce que** l'on utilise des ubiquinones ou des plastoquinones ayant de 0 à 12 motifs isoprène et éventuellement des radicaux alkyle.

6. Utilisation selon la revendication 1, **caractérisée en ce que** l'on utilise des ubiquinones ou des plastoquinones ayant 9 ou 10 motifs isoprène.

7. Utilisation selon la revendication 1, **caractérisée en ce que** l'on utilise conjointement des antioxydants, des substances de métabolisme énergétique cellulaire aérobie et/ou des agents absorbant les UV.

8. Utilisation selon la revendication 1, **caractérisée en ce que** les formulations topiques sont des formulations cosmétiques ou dermatologiques, en particulier des, huiles, des émulsions E/H, H/E ou E/H/E.
